# EUROPEAN PATENT SPECIFICATION

(11) **EP 4 008 297 B1**
(45) Date of publication and mention of the grant of the patent: **23.07.2025**
(21) Application number: 21211831.9
(22) Date of filing: 01.12.2021
(51) Int. Cl.: A61F 9/007, A61M 5/178, A61M 5/31, A61M 5/34

(54) **GRIP FOR MOUNTING A CYSTOTOME TO A SYRINGE, AN ASSEMBLY THEREOF, AND A METHOD RELATED THERETO**
GRIFF ZUR BEFESTIGUNG EINES CYSTOTOMS AN EINER SPRITZE, ANORDNUNG DAVON UND VERFAHREN IM ZUSAMMENHANG DAMIT
POIGNÉE POUR FIXER UN CYSTOTOME SUR UNE SERINGUE, ENSEMBLE ASSOCIÉ ET PROCÉDÉ ASSOCIÉ

(30) Priority: 03.12.2020 US 202063120872 P
(43) Date of publication of application: 08.06.2022
(73) Proprietor: Beaver-Visitec International (US), Inc., Waltham, MA 02451 (US)
(72) Inventor: WANG, Paula Y., Sudbury, MA 10776 (US)
(74) Representative: Rummler, Felix

(56) References cited:
- EP-A1- 1 192 965
- WO-A1-2020/014292
- WO-A1-2020/086332
- US-A- 4 369 781
- US-A- 5 167 618
- US-A- 5 496 281
- US-A1- 2002 173 753
- US-A1- 2004 116 950
- US-A1- 2013 131 606
- US-A1- 2018 021 052
- US-A1- 2018 125 711
- US-A1- 2020 155 770
- US-B1- 6 306 155

## Description

### Field of the Invention

The subject invention relates to syringe assemblies used in ophthalmic surgery, particularly, in performing capsulotomies.

### Background of the Invention

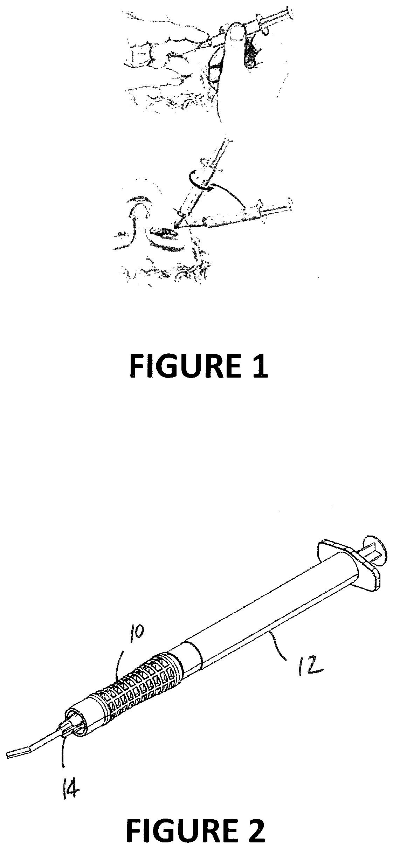

Cystotomes are known in the prior art for incising the lens capsule of a patient's eye in performing a capsulotomy, e.g., during capsulorhexis., as shown in Figure 1. Examples of commercially-available cystotomes are irrigating cystotomes sold by Beaver-Visitec International, Inc. of Waltham, MA. A cystotome is typically provided with a plastic hub from which the cystotome extends distally. The distal end of the cystotome defines an opening with a radially-protruding sharpened edge for incising the patient's eye, e.g., incising the anterior capsule of the patient's eye in performing a capsulotomy, and, the hub includes a female luer fitting for mounting onto a standard syringe. In use, the syringe allows for introduction of irrigation fluid through the cystotome during a capsulotomy.

Typically, a practitioner grips the syringe to manipulate, e.g., rotate, the cystotome during a capsulotomy, often adjusting their grip especially for a continuous curvilinear capsulorhexis (CCC). Standard syringe barrels typically have smooth outer surfaces and a constant-diameter barrel, providing no grip enhancement or ergonomic optimization, since grip enhancement is not a factor in administering injections.

US 4 369 781 A describes a luer connector pertaining to the features of the preamble of claim 1.

US 2013/131606 A1 describes a syringe including a slide member, wherein force exerted on the slide member causes movement of a syringe plunger within a hollow or partially hollow syringe barrel, for withdrawing fluid from a source into the syringe, and also includes a reusable adaptor for use with existing conventional syringes, which has a ring that wholly or partially encircles the barrel and indirectly attaches to a plunger flange.

WO 2020/014292 A1 describes an injector for delivering medicament including a first sleeve defining a longitudinal axis, a medicament container, and a locking tab. The medicament container is disposed within the first sleeve and configured for axial translation with respect to the first sleeve along the longitudinal axis. The medicament container includes a barrel, a needle mounted to a distal end of the barrel, a seal slidably mounted in the barrel, and a plunger rod. The locking tab extends through an aperture in the first sleeve and is in contact with or adjacent to a portion of the medicament container such that the locking tab restricts translation of the barrel with respect to the first sleeve along the longitudinal axis. The locking tab is configured to be movable to a second position in which the locking tab does not restrict translation of the barrel.

US 2004/116950 A1 describes an instrument and method for creating an intraocular incision.

### Summary of the Invention

The present invention is defined in the independent claims. The dependent claims recite selected optional features.

In the following, each of the described methods, apparatuses, examples, and aspects, which do not fully correspond to the invention as defined in the claims is thus not according to the invention and is, as well as the whole following description, present for illustration purposes only or to highlight specific aspects or features of the claims.

In one aspect of the subject invention, a syringe assembly for performing a capsulotomy on a patient's eye with a cystotome is provided, the assembly comprising: a syringe having a luer tip with a liquid outlet at a distal end thereof; and, a grip including an elongated body extending along a longitudinal axis (LA) between first and second ends, the first end defining a female luer fitting configured for mounting onto the luer tip of the syringe, an inlet opening being defined in the female luer fitting, the second end defining a male luer fitting defining an outlet opening at a distal end thereof, and, a liquid passageway defined through the body between the inlet opening and the outlet opening to allow liquid flow therebetween, wherein, the body defines an outer surface encircling the longitudinal axis (LA), the outer surface being at least partially textured, wherein the grip is mounted to the syringe with the female luer fitting mounted to the luer tip, wherein the inlet opening being aligned with the liquid outlet of the luer tip, and a cystotome, distally extending from a hub, the cystotome configured for incising the lens capsule of a patient's eye, the hub having a secondary female luer fitting mounted to the male luer fitting.

In a further example, a method is provided for performing a capsulotomy on a patient's eye, the method including: providing a grip having an elongated body extending along a longitudinal axis between first and second ends, the first end defining a female luer fitting with an inlet opening defined therein, the second end defining a male luer fitting defining an outlet opening at a distal end thereof, and, a liquid passageway defined through the body between the inlet opening and the outlet opening to allow liquid flow therebetween, wherein, the body defines an outer surface encircling the longitudinal axis, the outer surface being at least partially textured; mounting the female luer fitting of the grip onto a luer tip of a syringe containing irrigation fluid; and, mounting a hub, from which distally extends a cystotome, onto the male luer fitting of the grip.

As used herein, the term "distal," and derivatives thereof (e.g., distally) refers to a direction towards or closer to a patient. As used herein, the term "proximal," and derivates thereof (e.g., proximally) refers to a direction away or farther from a patient.

These and other features of the subject invention will be better understood through a study of the following detailed description.

### Brief Description of the Drawings

Figure 1 depicts the use of a cystotome in performing a capsulotomy, the cystotome being mounted to a standard syringe as known in the prior art;
Figure 2 is a perspective view of a syringe assembly in accordance with the subject invention;
Figure 3 is side view of the syringe assembly of Figure 2;
Figure 4 is an exploded view of syringe assembly of Figure 2;
Figure 5 is a cross-sectional view of a syringe useable with the subject invention; and,
Figures 6-14 are various views of grips useable with the subject invention.

### Detailed Description of the Invention

With reference to the Figures, a grip 10 is shown in accordance with the subject invention. The grip 10 is mountable to a syringe 12 and is configured to mountingly receive a cystotome 14.

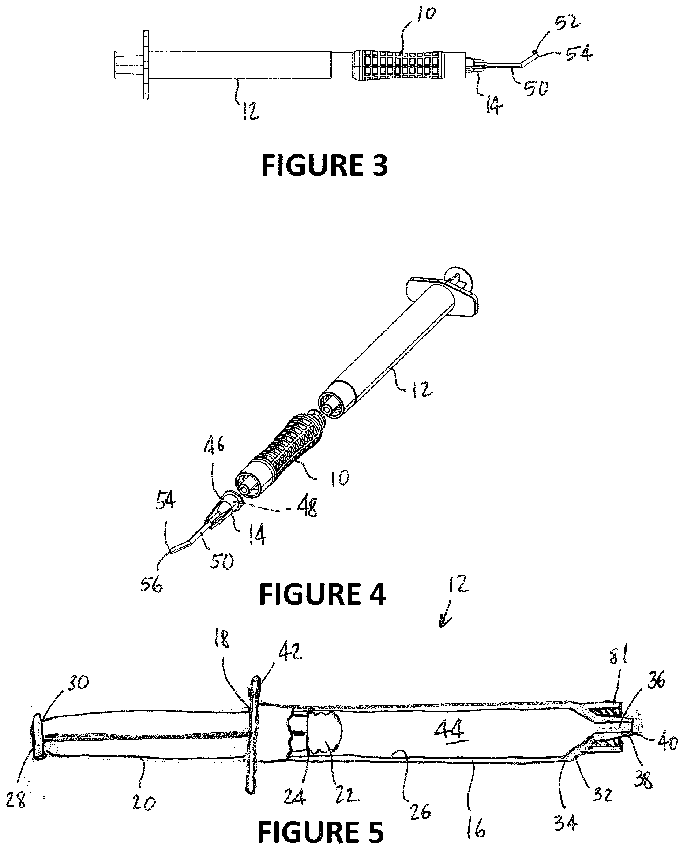

As shown in Figure 5, the syringe 12 may be any standard syringe, including being pre-fillable (e.g., glass barrel) or disposable (e.g., plastic barrel). The syringe 12 may include a barrel 16, formed of glass and/or plastic, for accommodating a liquid, which has an open proximal end 18, through which plunger 20 may extend. A piston 22, which may be elastomeric, may be provided at a distal end 24 of the plunger 20, the piston 22 formed for sliding, liquid-tight engagement with inner surface 26 of the barrel 16. A thumb pad 28 may be provided at a proximal end 30 of the plunger 20. A wall 32 may extend across a distal end 34 of the barrel 16. The wall 32 may be tapered to direct flow to a liquid outlet 36. The wall 32 may be configured to define a luer tip 38, as is known in the art. The luer tip 38 may be a slip tip or provided with a surrounding internally-threaded collar to provide a "luer lock" arrangement. The liquid outlet 36 extends through a distal end 40 of the luer tip 38. For ease of handling, one or more finger flanges 42 may protrude radially outwardly from the barrel 16 about the open proximal end 18.

The barrel 16 defines a reservoir 44 distally of the plunger 20. Distal movement of the plunger 20, results in distal movement of the piston 22 and, thus, liquid contained in the reservoir 44 being urged through the liquid outlet 36.

The cystotome 14 may be of any known type. By way of non-limiting example, the cystotome 14 may include a hub 46, which may be plastic, having a secondary female luer fitting 48 defined therein. The cystotome 14 may be in the form of a cannula 50, which may be bent, and secured to the hub 46 to extend distally therefrom. The cystotome 14 may be configured for incising a lens capsule of a patient's eye, e.g., by including one or more radially protruding cutting edge(s) 52 at or proximate to a distal end 54 of the cannula 50. The cystotome 14 may include a distal opening 56 formed at the distal end 54, through which irrigation fluid may flow.

The grip 10 includes a body 58, which may be a unitary piece, prepared by various techniques e.g., by molding or forming (e.g., three-dimensional printing). The grip 10 may be formed of thermoplastic and/or elastomeric material. Material must be selected to allow the grip 10 to be sterilized (e.g., by UV exposure, gas exposure, steam exposure, and so forth).

The body 58 is elongated to extend along a longitudinal axis LA between first and second ends 60, 62 thereof. The first end 60 defines a female luer fitting 64 configured for mounting onto the luer tip 38 of the syringe 12. The female luer fitting 64 defines an inlet opening 66, which is formed to align with the liquid outlet 36 of the luer tip 38 with the female luer fitting 64 being mounted to the luer tip 38. The second end 62 defines a male luer fitting 68 which defines an outlet opening 70 at a distal end 72 thereof. A liquid passageway 74 may be defined through the body 58 between the inlet opening 66 and the outlet opening 70 to allow liquid flow therebetween. The liquid passageway 74 preferably is straight, to run along the longitudinal axis LA. Changes in direction are not desired since such may cause resistance to flow through the liquid passageway 74. It is also preferred that the liquid passageway 74 generally have a constant cross-section. The liquid passageway 74 may taper in a direction from the inlet opening 66 to the outlet opening 70, preferably, the taper being gradual.

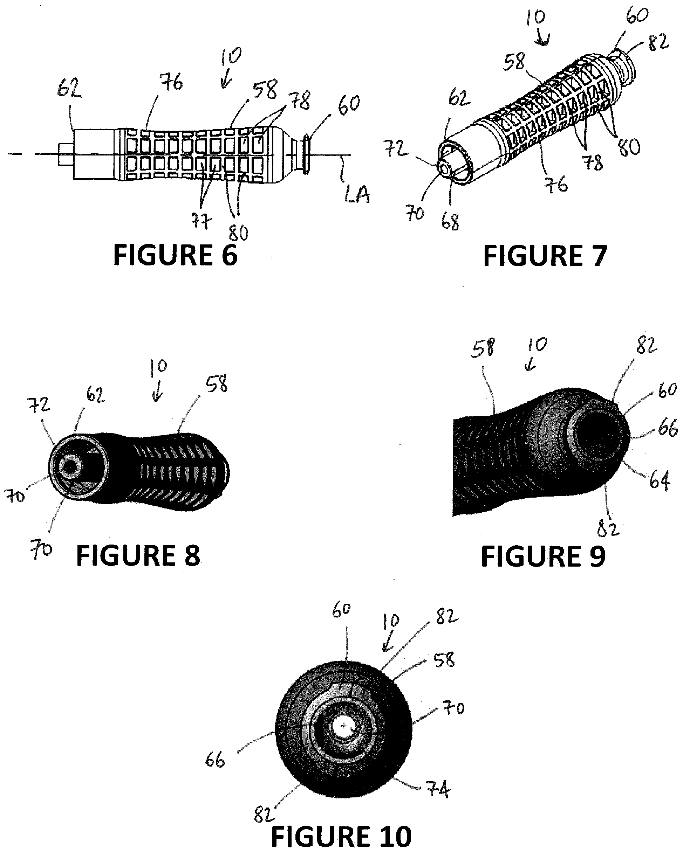

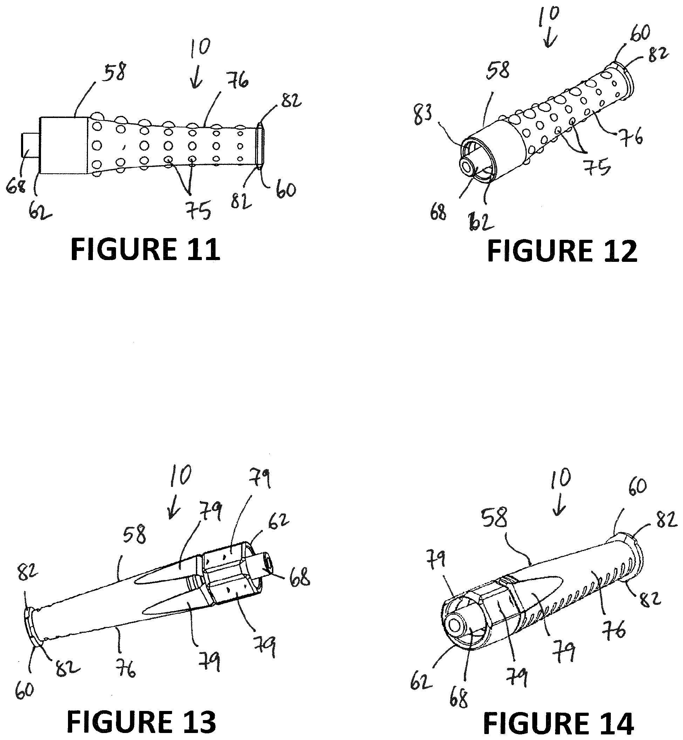

The body 58 defines an outer surface 76 which encircles the longitudinal axis LA. The outer surface 76 is at least partially textured, to enhance grip therewith. A full circumference of the body 58 may be textured, e.g., to define an annular band of texturing, to provide a practitioner with a radially uninterrupted textured surface for rotational adjustment of the cystotome 14. The texturing may be provided by any known arrangement, including, but not limited to, raised elements 75 (such as beads and/or ribs) and/or hollows 77 (such as channels or dimples). Figures 11 and 12 show exemplary forms of the raised elements 75, particularly as raised beads. The raised elements 75 may include regular or irregular patterns of protruding or embossed elements. Likewise, the hollows 77may include regular or irregular patterns of recesses or indentations. For example, as shown in Figure 7, the hollows 77 may include spaced-apart discontinuities 78 defined in the outer surface 76 where open pockets are defined between solid portions of the outer surface 76. The discontinuities 78 may be defined by a plurality of intersecting ribs 80. In addition, or alternatively, the outer surface 76 may be defined by an anti-slip material which provides enhanced gripping. Examples of suitable anti-slip materials include one or more of a polymeric foam, a polymeric mesh, an elastomer, and a coated or treated polymer. The body 58 may be provided as a two-part piece with a core of one material encased by the outer surface 76 of a different material. In this configuration, the core may be formed as a unitary piece with the outer surface 76 being applied thereto as a secondary operation. The core may contain the female luer fitting 64, the male luer fitting 68, and the liquid passageway 74.

In addition, or alternatively, the body 58 may be configured to enhance handling. For example, as shown in Figure 6, the diameter of the body 58 may vary along the longitudinal axis LA. This allows for the body 58 to have a concave shape, with indentations in which portions of a practitioner's fingers may nest. Other shapes are possible such as one or more undulations, convexities, and so forth. In addition, as shown in Figures 13-14, portions of the body 58 may be flattened or otherwise configured to define one or more panels or truncations 79.

The grip 10 may be mounted to the syringe 12 with the female luer fitting 64 being mounted to the luer tip 38. One or more thread elements 82 may be provided at the first end 60, e.g., about the female luer fitting 64, configured to engage, e.g., threadedly engage, a threaded collar 81 if provided about the luer tip 38 to form a "luer lock." Slip mounting alone may be utilized. In addition, the cystotome 14 may be mounted to the grip 10 with the secondary female luer fitting 48 being mounted to the male luer fitting 68. An internally-threaded collar 83 may be provided about the male luer fitting 68, as shown in Figure 12, with one or more cooperating thread elements provided on the hub 46, about the secondary female luer fitting 48, to allow a "luer lock" to form therebetween. Slip mounting alone may be also utilized at this interface.

The grip 10, the syringe 12, and the cystotome 14 collectively form a syringe assembly useable for performing a capsulotomy on a patient's eye. The grip 10 provides a practitioner with an enhanced grip surface distally of the syringe 12, to allow for good handling. Many practitioners use a rolling motion of a cystotome in performing a capsulotomy. With the outer surface 76 being textured, the practitioner is provided with an improved grip surface. With the outer surface 76 being textured about its entire circumference, an improved grip surface may be provided agnostic to rotational orientation of the grip 10.

In use, an assembly for performing a capsulotomy on a patient's eye may be prepared by providing the grip 10. The grip 10 is required to be sterilized for use, as well as, the syringe 12 and the cystotome 14. The grip 10 is mounted to the syringe 12 and the cystotome 14 is mounted to the grip 10, e.g., via the hub 46. With this arrangement, the syringe 12 may be provided with irrigation fluid that may be dispensed through the cystotome 14 during a capsulotomy.

## Claims

1. A syringe assembly for performing a capsulotomy on a patient's eye with a cystotome, the assembly comprising:
a syringe (12) having a luer tip (38) with a liquid outlet (36) at a distal end (40) thereof; and,
a grip (10) including an elongated body (58) extending along a longitudinal axis (LA) between first and second ends (60, 62), the first end (60) defining a female luer fitting (64) configured for mounting onto the luer tip (38) of the syringe (12), an inlet opening (66) being defined in the female luer fitting (64), the second end (62) defining a male luer fitting (68) defining an outlet opening (70) at a distal end (72) thereof, and, a liquid passageway (74) defined through the body (58) between the inlet opening (66) and the outlet opening (70) to allow liquid flow therebetween, wherein, the body (58) defines an outer surface (76) encircling the longitudinal axis (LA), the outer surface (76) being at least partially textured,
wherein the grip (10) is mounted to the syringe (12) with the female luer fitting (64) mounted to the luer tip (38),
and
the inlet opening (66) being aligned with the liquid outlet (36) of the luer tip (38),
**characterized by**
a cystotome (14), distally extending from a hub (46), the cystotome (14) configured for incising the lens capsule of a patient's eye, the hub (46) having a secondary female luer fitting (48) mounted to the male luer fitting (68).

2. The syringe assembly of claim 1, wherein the diameter of the body (58) varies along the longitudinal axis (LA).

3. The syringe assembly of claim 2, wherein the body (58) defines a concave shape.

4. The syringe assembly of any preceding claim, wherein the outer surface (76) is textured by a plurality of spaced-apart discontinuities (78) and/or raised elements (75).

5. The syringe assembly of claim 4, wherein the outer surface (76) includes a plurality of intersecting ribs (80) defining the spaced-apart discontinuities (78).

6. The syringe assembly of any preceding claim, further comprising an internally-threaded collar (83) disposed about the male luer fitting (68).

7. The syringe assembly of any preceding claim, further comprising at least one thread (82) protruding radially outwardly from the female luer fitting (48).

8. The syringe assembly of any preceding claim, wherein the outer surface (76) is textured about a full circumference of the body (58).

9. The syringe assembly of any preceding claim, wherein the syringe (12) contains irrigation fluid.

10. A method of preparing an assembly for performing a capsulotomy on a patient's eye, the method comprising:
providing a grip having an elongated body extending along a longitudinal axis between first and second ends, the first end defining a female luer fitting with an inlet opening defined therein, the second end defining a male luer fitting defining an outlet opening at a distal end thereof, and, a liquid passageway defined through the body between the inlet opening and the outlet opening to allow liquid flow therebetween, wherein, the body defines an outer surface encircling the longitudinal axis, the outer surface being at least partially textured;
mounting the female luer fitting of the grip onto a luer tip of a syringe containing irrigation fluid; and,
mounting a hub, from which distally extends a cystotome, onto the male luer fitting of the grip.

11. The method of claim 10, wherein the outer surface is textured about a full circumference of the body.

## Patentansprüche

1. Spritzenbaugruppe für das Durchführen einer Kapsulotomie mit einem Cystotom an einem Auge eines Patienten, die Folgendes umfasst:
eine Spritze (12), die einen Luer-Konus (38) mit einem Flüssigkeitsauslass (36) an seinem distalen Ende (40) aufweist, und
einen Griff (10) mit einem länglichen Hauptteil (58), der sich entlang einer Längsachse (LA) zwischen einem ersten und einem zweiten Ende (60, 62) erstreckt, wobei das erste Ende (60) einen weiblichen Luer-Anschluss (64) definiert, der zum Anbringen an dem Luer-Konus (38) der Spritze (12) konfiguriert ist, wobei in dem weiblichen Luer-Anschluss (64) eine Einlassöffnung (66) definiert ist, und das zweite Ende (62) einen männlichen Luer-Anschluss (68) definiert, der an seinem distalen Ende (72) eine Auslassöffnung (70) definiert, und mit einem zwischen der Einlassöffnung (66) und der Auslassöffnung (70) in dem Hauptteil (58) definierten Flüssigkeitsdurchgang (74), so dass Flüssigkeit dazwischen strömen kann, wobei der Hauptteil (58) eine Außenfläche (76) definiert, die die Längsachse (LA) umgibt, wobei die Außenfläche (76) zumindest teilweise strukturiert ist,
wobei der Griff (10) mit dem am Luer-Konus (38) angebrachten weiblichen Luer-Anschluss (64) an der Spritze (12) angebracht ist und
die Einlassöffnung (66) auf den Flüssigkeitsauslass (36) des Luer-Konus (38) ausgerichtet ist,
**gekennzeichnet durch**
ein Cystotom (14), das sich von einer Nabe (46) aus distal erstreckt, wobei das Cystotom (14) zum Einschneiden der Linsenkapsel eines Auges eines Patienten konfiguriert ist, wobei die Nabe (46) einen sekundären weiblichen Luer-Anschluss (48) aufweist, der an dem männlichen Luer-Anschluss (68) angebracht ist.

2. Spritzenbaugruppe nach Anspruch 1, wobei der Durchmesser des Hauptteils (58) entlang der Längsachse (LA) variiert.

3. Spritzenbaugruppe nach Anspruch 2, wobei der Hauptteil (58) eine konkave Form definiert.

4. Spritzenbaugruppe nach einem der vorhergehenden Ansprüche, wobei die Außenfläche (76) mit mehreren beabstandeten Unregelmäßigkeiten (78) und/oder erhabenen Elementen (75) strukturiert ist.

5. Spritzenbaugruppe nach Anspruch 4, wobei die Außenfläche (76) mehrere sich überschneidende Rippen (80) aufweist, die die beabstandeten Unregelmäßigkeiten (78) definieren.

6. Spritzenbaugruppe nach einem der vorhergehenden Ansprüche, die ferner einen mit Innengewinde versehenen Ring (83) umfasst, der um den männlichen Luer-Anschluss (68) herum angeordnet ist.

7. Spritzenbaugruppe nach einem der vorhergehenden Ansprüche, die ferner mindestens ein Gewinde (82) umfasst, das von dem weiblichen Luer-Anschluss (48) aus radial nach außen vorsteht.

8. Spritzenbaugruppe nach einem der vorhergehenden Ansprüche, wobei die Außenfläche (76) um einen gesamten Umfang des Hauptteils (58) herum strukturiert ist.

9. Spritzenbaugruppe nach einem der vorhergehenden Ansprüche, wobei die Spritze (12) Spülfluid enthält.

10. Verfahren zum Vorbereiten einer Baugruppe für das Durchführen einer Kapsulotomie an einem Auge eines Patienten, das Folgendes umfasst:
Bereitstellen eines Griffs mit einem länglichen Hauptteil, der sich entlang einer Längsachse zwischen einem ersten und einem zweiten Ende erstreckt, wobei das erste Ende einen weiblichen Luer-Anschluss definiert, in dem eine Einlassöffnung definiert ist, und das zweite Ende einen männlichen Luer-Anschluss definiert, der an seinem distalen Ende eine Auslassöffnung definiert, und mit einem zwischen der Einlassöffnung und der Auslassöffnung in dem Hauptteil definierten Flüssigkeitsdurchgang, so dass Flüssigkeit dazwischen strömen kann, wobei der Hauptteil eine Außenfläche definiert, die die Längsachse umgibt, wobei die Außenfläche zumindest teilweise strukturiert ist,
Anbringen des weiblichen Luer-Anschlusses des Griffs an einem Luer-Konus einer Spritze, die Spülfluid enthält, und
Anbringen einer Nabe, von der aus sich ein Cystotom distal erstreckt, an dem männlichen Luer-Anschluss des Griffs.

11. Verfahren nach Anspruch 10, wobei die Außenfläche um einen gesamten Umfang des Hauptteils herum strukturiert ist.

## Revendications

1. Ensemble formant seringue servant à réaliser une capsulotomie sur l'œil d'un patient au moyen d'un cystotome, l'ensemble comportant :
une seringue (12) ayant un embout Luer (38) avec une sortie de liquide (36) au niveau d'une extrémité distale (40) de celle-ci ; et,
un élément de préhension (10) comprenant un corps allongé (58) s'étendant le long d'un axe longitudinal (LA) entre des première et deuxième extrémités (60, 62), la première extrémité (60) définissant un raccord Luer femelle (64) configuré à des fins de montage sur l'embout Luer (38) de la seringue (12), une ouverture d'entrée (66) étant définie dans le raccord Luer femelle (64), la deuxième extrémité (62) définissant un raccord Luer mâle (68) définissant une ouverture de sortie (70) au niveau d'une extrémité distale (72) de celui-ci, et, une voie de passage de liquide (74) définie au travers du corps (58) entre l'ouverture d'entrée (66) et l'ouverture de sortie (70) pour permettre au liquide de s'écouler entre elles, dans lequel le corps (58) définit une surface extérieure (76) encerclant l'axe longitudinal (LA), la surface extérieure (76) étant au moins partiellement texturée,
dans lequel l'élément de préhension (10) est monté sur la seringue (12) avec le raccord Luer femelle (64) monté sur l'embout Luer (38), et,
l'ouverture d'entrée (66) étant alignée sur la sortie de liquide (36) de l'embout Luer (38), **caractérisé par**
un cystotome (14), s'étendant de manière distale depuis un collet (46), le cystotome (14) étant configuré pour inciser la capsule du cristallin de l'œil d'un patient, le collet (46) ayant un raccord Luer femelle secondaire (48) monté sur le raccord Luer mâle (68).

2. Ensemble formant seringue selon la revendication 1, dans lequel le diamètre du corps (58) varie le long de l'axe longitudinal (LA).

3. Ensemble formant seringue selon la revendication 2, dans lequel le corps (58) définit une forme concave.

4. Ensemble formant seringue selon l'une quelconque des revendications précédentes, dans lequel la surface extérieure (76) est texturée par une pluralité de discontinuités espacées (78) et/ou d'éléments en relief (75).

5. Ensemble formant seringue selon la revendication 4, dans lequel la surface extérieure (76) comprend une pluralité de nervures d'intersection (80) définissant les discontinuités espacées (78).

6. Ensemble formant seringue selon l'une quelconque des revendications précédentes, comportant par ailleurs un collier à filetage interne (83) disposé autour du raccord Luer mâle (68).

7. Ensemble formant seringue selon l'une quelconque des revendications précédentes, comportant par ailleurs au moins un filetage (82) faisant saillie radialement vers l'extérieur depuis le raccord Luer femelle (48).

8. Ensemble formant seringue selon l'une quelconque des revendications précédentes, dans lequel la surface extérieure (76) est texturée autour de toute une circonférence du corps (58).

9. Ensemble formant seringue selon l'une quelconque des revendications précédentes, dans lequel la seringue (12) contient un fluide d'irrigation.

10. Procédé de préparation d'un ensemble servant à réaliser une capsulotomie sur l'œil d'un patient, le procédé comportant les étapes consistant à :
fournir un élément de préhension ayant un corps allongé s'étendant le long d'un axe longitudinal entre des première et deuxième extrémités, la première extrémité définissant un raccord Luer femelle avec une ouverture d'entrée définie dans celui-ci, la deuxième extrémité définissant un raccord Luer mâle définissant une ouverture de sortie au niveau d'une extrémité distale de celui-ci, et, une voie de passage de liquide définie au travers du corps entre l'ouverture d'entrée et l'ouverture de sortie pour permettre au liquide de s'écouler entre elles, dans lequel le corps définit une surface extérieure encerclant l'axe longitudinal, la surface extérieure étant au moins partiellement texturée ;
monter le raccord Luer femelle de l'élément de préhension sur un embout Luer d'une seringue contenant du liquide d'irrigation ; et,
monter un collet, à partir duquel s'étend de manière distale un cystotome, sur le raccord Luer mâle de l'élément de préhension.

11. Procédé selon la revendication 10, dans lequel la surface extérieure est texturée sur toute une circonférence du corps.
